# EUROPÄISCHE PATENTSCHRIFT

(11) **EP 1 011 883 B1**
(45) Veröffentlichungstag und Bekanntmachung des Hinweises auf die Patenterteilung: **07.08.2002**
(21) Anmeldenummer: 98947474.7
(22) Anmeldetag: 29.08.1998
(51) Int. Cl.: B09C 1/10

(54) **VERFAHREN ZUR VEREINFACHTEN BIOLOGISCHEN SANIERUNG VON BODENFLÄCHEN, DIE MIT MINERALÖL-BASIERTEN ALTLASTEN KONTAMINIERT SIND**
SIMPLIFIED METHOD FOR BIOLOGICALLY CLEANING AREAS OF SOIL CONTAMINATED WITH LEFTOVER POLLUTANT MINERAL OIL-BASED MATERIALS
PROCEDE D'ASSAINISSEMENT BIOLOGIQUE SIMPLIFIE DE SOLS CONTAMINES PAR DES MATIERES USEES A BASE D'HUILE MINERALE

(30) Priorität: 08.09.1997 DE 19739071
(43) Veröffentlichungstag der Anmeldung: 28.06.2000
(73) Patentinhaber: Cognis Deutschland GmbH & Co. KG, 40589 Düsseldorf (DE); INDUSTRIEANLAGEN-BETRIEBSGESELLSCHAFT M.B.H., 85521 Ottobrunn (DE)
(72) Erfinder: KOPP-HOLTWIESCHE, Bettina, D-40599 Düsseldorf (DE); LAUER, Ursula, D-81739 München (DE)
(86) Internationale Anmeldenummer: EP9805478
(87) Internationale Veröffentlichungsnummer: WO9912668

(56) Entgegenhaltungen:
- WO-A-93/06051
- DE-A- 3 812 364
- US-A- 3 616 204

## Beschreibung

Der jahrzehntelange sorglose Umgang mit Mineralölen, Mineralölfraktionen und daraus abgeleiteten Produkten im steigenden Ausmaß ihrer wirtschaftlichen Verwertung hat - nach heutigem Wissen und Verständnis - weltweit zu beträchtlichen Schädigungen geführt, die heute auch als Altlasten bezeichnet werden. Ein großer Bereich dieser Altlasten auf Basis von Mineralöl-basierten Kohlenwasserstoffverbindungen - im nachfolgenden der Einfachheit halber als "MKW" bezeichnet - erfaßt Bodenflächen, an denen beispielsweise über Jahrzehnte hinweg entsprechende MKW's gelagert, umgepumpt, verteilt und/oder transportiert wurden. Lediglich beispielhaft seien hier entsprechende Tanklager für die industrielle Verwertung der MKW's und/oder Rohleitungssysteme für den gegebenenfalls auch sehr weit führenden Transport von MKW's benannt.

Seit geraumer Zeit ist in zunehmenden Ausmaß das Verständnis für das hier betroffene verantwortungslose Handeln aus der Vergangenheit erwachsen. Einerseits hat das - wenigstens in wichtigen Industriestaaten der Welt - zu sehr viel strengeren Anforderungen an das schadstofffreie Handling der hier betroffenen Stoffklasse geführt. Zum anderen ist ein großer und umfangreicher praktischer und druckschriftlicher Stand der Technik zur Beseitigung aufgetretener Schäden, insbesondere entsprechender Altlasten entwickelt worden. Trotz dieses in der Zwischenzeit verschärften Bildes der Bedeutung der hier angesprochenen Umweltproblematik und trotz substantieller Erhöhung der Sicherheit im Umgang mit MKW's besteht auch noch heute nach wie vor dringender Bedarf zur kostengünstigen und einfachen regenarativen Aufarbeitung weiter Flächenbereiche des Erdbodens, die insbesondere mit entsprechenden Altlasten derart stark belastet sind, daß eine einfache Umwidmung dieser betroffenen Bereiche durch behördliche Anordnung nicht möglich ist. Es bedarf zunächst einer hinreichenden Reinigung dieser von Schadstoffen beladenen Flächen bis in hinreichende Tiefen des Erdbodens, um neue Nutzungsmöglichkeiten für diese Flächenbereiche zu erschließen.

Literatur und Praxis kennen zahlreiche Vorschläge zur Lösung der hier angeschnittenen Problemstellung. Die Effektivität der jeweils eingesetzten Reinigungsmethodik ist verständlicherweise ein entscheidender Faktor für die Anwendbarkeit der jeweiligen Methode. Sie ist aber nicht der alleinbestimmende Faktor. Gerade bei der Regenerierung größerer Erdreichflächen spielt der einzusetzende Kostenaufwand mindestens eine gleichgroße Rolle.

Die Bekämpfung von Verschmutzungen auf Basis von Kohlenwasserstoffverbindungen - beispielsweise MKW - in Böden und Gewässern mittels Bioremediation gewinnt zunehmende Bedeutung. Kohlenwasserstoffverbindungen verzehrende Mikroorganismen sind im Rahmen dieser Technologie wertvolle Arbeitsmittel, soweit es gelingt, ihre Anreicherung und/oder ihr Wachstum am Ort der Verschmutzung hinreichend zu stimulieren. Aus dem neueren Schrifttum sei verwiesen auf die Veröffentlichungen in Chemische Industrie 5/91, 10 - 12 "Hunger auf Erdöl" sowie in Erdöl und Kohle - Erdgas, 44, April 1991, 197 - 200, Th. Höpner et al. "Die Ölkatastrophe im Persisch-Arabischen Golf' sowie die dort zitierte umfangreiche Literatur.

Das Arbeitsprinzip der Bioremediation sieht die optimale Förderung des Wachstums der Schmutz-verzehrenden Mikroorganismenpopulationen vor. Nach den Angaben des Standes der Technik - vgl. beispielsweise DE-A-42 18 243 und PC/EP 92/02146 - stehen hier zwei wichtige Hilfsmittel im Vordergrund: Zum einen bedarf es der Zufuhr wachstumsfördernder Elemente, die in der Regel im Verschmutzungsbereich in nicht hinreichenden Konzentrationen zur Verfügung stehen. Hierbei handelt es sich in erster Linie um anorganische und/oder organische Verbindungen des Stickstoffs und des Phosphors, die als Nährstoffkonzentrate zur Stimulation und als Wachstumshilfe für die beschleunigte An- und Aufzucht der Kohlenwasserstoffverbindungen-verzehrenden Mikroorganismen angeboten werden. Zum anderen werden nach diesen Angaben des Standes der Technik häufig - insbesondere zur Beschleunigung des biologischen Abbaus in der Anfangsphase - vorgebildete Konzentrate geeigneter Kohlenwasserstoff-verzehrender Mikroorganismen eingesetzt, die beispielsweise im wöchentlichen Abstand auf die verschmutzten Bereiche aufgetragen werden können. Je nach der gegebenen Situation, insbesondere der individuellen Vorgeschichte der reinigungsbedürftigen Gebiete, kann allerdings gegebenenfalls auch davon ausgegangen werden, daß ein solches Animpfen mit Mikroorganismen-Konzentraten unnötig ist. in der Regel wird das immer der Fall sein, wenn der natürliche Vorgang des biologischen Abbaus bereits zur Ausbildung hinreichender Konzentrationen von Mikroorganismenstämmen geführt hat, vgl. in diesem Zusammenhang beispielsweise die zweite der zuvor zitierten Literaturstellen.

Die erfindungsgemäße Lehre geht von der Tatsache aus, daß insbesondere im Bereich der sogenannten Altlasten der im Prinzip mögliche natürliche Abbauprozeß durch Remediation unter den vor Ort gegebenen Bedingungen von Bodenbeschaffenheit, Klima und dergleichen nicht oder praktisch nicht stattfindet. Die erfindungsgemäße Lehre geht weiterhin von der Aufgabe aus, auch und gerade unter den hier betroffenen Voraussetzungen die Reinigung des Erdbodens durch Remediation bis in substantielle Tiefen der betroffenen Erdbodenschichten vornehmen zu können, ohne daß hierzu arbeitsaufwendige und dementsprechend kostspielige Maßnahmen wie das bekannte Auskoffern der betroffenen Bodenbereiche und deren Behandlung in einer aufgeschütteten Miete unter optimierten klimatischen Bedingungen nötig sind.

Bevor auf die Maßnahmen der erfindungsgemäßen Lehre zur Lösung dieser Aufgabenstellung nachfolgend eingegangen wird, sei auf eine Besonderheit verwiesen, die im Zusammenhang mit dem Verständnis der erfindungsgemäßen Lehre und der Anwendung der erfindungsgemäßen Maßnahmen in unmittelbarem Zusammenhang steht: Getrennt voneinander und in vertikal zunehmender Tiefe entnommene Bodenproben solcher typischen Altlastbereiche zeigen häufig ein Schadstoffbild, das offenbar charakteristisch für den hier betroffenen Verschmutzungsbereich ist. Besonders langkettige und/oder cyklische Kohlenwasserstoffverbindungen hohen Molekulargewichts, die sich durch besonders schwere Abbaubarkeit auszeichnen, finden sich in den oberen Schichten des Bodenbereiches angereichert. Es schließen sich dann - je nach Kornstruktur der betroffenen Bodenbereiche - gegebenenfalls beträchtliche Tiefenbereiche an, die eine offenbar leichter migrierende Schadstoffbelastung aufweisen. Auch im Bereich des Grundwasserpegels können beträchtliche Konzentrationen der unerwünschten Schadstoffbelastungen vorliegen. Diese Kohlenwasserstoffverbindungen sind offensichtlich von Molekülstruktur und -größe auch über längere Wanderungszeiträume befähigt, durch die Bodenstruktur hindurch zu wandern. Sie schwimmen dann gewissermaßen auf der Oberfläche des Grundwassers auf und werden hier im Laufe der Jahrzehnte an den Oberflächen der Bodenpartikel verfestigt, soweit sie nicht über das Grundwasser ausgetragen werden. Interessant, letztlich aber auch verständlich, ist die nachträgliche Deutung der unzureichenden Sanierung dieser durch Altlasten betroffenen Erdreichflächen: Die durch die Kohlenwasserstoffstruktur bedingte stark behinderte Abbaubarkeit der vergleichsweise großen KW-Moleküle in den oberen Flächen des Erdbereiches verhindert die natürliche Ausbildung Kohlenwasserstoff-verzehrender Mikroorganismenstämme, obwohl hier noch vergleichsweise größere Mengen an Luftsauerstoff zur Förderung des Bakterienwachstums zur Verfügung stünden. Verstärkt wird diese Behinderung in der Regel durch Mangel an wesentlichen Nährstoffen, insbesondere P und N. Die vergleichsweise leichter migrierenden KW-Komponenten sind in solche Tiefen des Erdbodens gespült, daß hier schon ein beträchtliches Minderangebot an Sauerstoff sowie gegebenenfalls auch hier Mangel an wesentlichen Nährstoffkomponenten besteht.

Von diesen Grunderkenntnissen ausgehend schlägt die erfindungsgemäße Lehre jetzt erstmalig ein außerordentlich einfaches und damit preiswertes sowie auch für große Flächenbereiche geeignetes Aufarbeitungsverfahren zur Entsorgung von MKW-Verunreinigungen vor, wobei insbesondere Erdreichflächen praktisch beliebiger Größe gereinigt werden können, die mit entsprechenden Altlasten verunreinigt sind.

### Gegenstand der Erfindung

Erfindungsgegenstand ist dementsprechend ein Verfahren zur vereinfachten biologischen Sanierung von Erdbodenflächen praktisch beliebigen Ausmaßes, die mit Mineralöl-basierten Kohlenwasserstoffen (MKW) und dabei insbesondere entsprechenden Altlasten kontaminiert sind, durch Behandlung mit Nährstoffkonzentraten zur Stimulation und Wachstumshilfe für eine beschleunigte An- und Aufzucht von MKW-verzehrenden Mikroorganismen. Das erfindungsgemäße Verfahren ist dadurch gekennzeichnet, daß man
(a) nach einer vorgängigen Durchwässerung
(b1) in wenigstens einem Arbeitsgang einerseits die zu sanierende Erdbodenfläche tiefgängig auf- und umbricht,
(b2) und andererseits ebenfalls in wenigstens einem Arbeitsgang das Nährstoffkonzentrat in Form einer wäßrig verdünnten Emulsion und/oder Dispersion aufträgt und dabei insbesondere im wesentlichen gleichmäßig verteilt aufsprüht und
(c) abschließend intensiv nachwässert.

In einer bevorzugten Ausführungsform ist erfindungsgemäß vorgesehen, wenigstens die Arbeitsstufen (a), (b2) und (c) in größeren Zeitabständen zu wiederholen. Bevorzugte Zeitabstände liegen hier im Bereich von etwa 1 bis 6 Monaten. Dabei ist eine wenigstens 1- bis 3fache Wiederholung zweckmäßig.

In weiteren bevorzugten Ausführungsformen kann es erfindungsgemäß zweckmäßig sein zusätzlich Konzentrate von Mikroorganismen auf die Bodenfläche aufzutragen, die zum Abbau von MKW-Verbindungen befähigt sind. Hierbei kann der Einsatz entsprechender Mikroorganismen-Konzentrate natürlichen Ursprungs eine besonders wichtige Ausführungsform sein. Insbesondere zur Entsorgung auch vergleichsweise tiefgelegener Bodenschichten von dort vorliegenden MKW's sieht die erfindungsgemäße Lehre in einer besonderen Ausführungsform vor, entweder eine Zwangsbelüftung dieser tiefergelegenen Bodenformationen zusätzlich einzurichten und/oder den Eintrag chemisch gebundenen und im Erdboden freiwerdenden Sauerstoffs vorzusehen. Auf Einzelheiten hierzu wird im Nachfolgenden eingegangen.

### Einzelheiten zur erfindungsgemäßen Lehre

Die zuvor gegebene Definition der erfindungsgemäß vorgesehenen Arbeitsschritte macht verständlich, daß hier - nach Wissen der Anmelderin erstmalig - ein technisch derart unkomplizierter Ansatz zur Beseitigung der MKW durch Remediation vorgeschlagen wird, daß Flächenbereiche praktisch beliebiger Größe und praktisch beliebigen Ausmaßes der MKW-Verschmutzung nach Menge und Alter unter akzeptablen Arbeits- und Kostenbedingungen der biologischen Sanierung unterworfen werden können. Im einzelnen gilt:

Das zu sanierende Erdreichgebiet soll hinreichend befeuchtet bzw. durchnässt sein. Dieser vorbehandelnde Arbeitsschritt kann Gebrauch von dem natürlichen Prozeß des Regenfalls als auch von der einfachen Methode der künstlichen Bewässerung machen. Die in der Regel durch jahrzehntelange mechanische Belastung verdichtete Erdreichoberfläche soll in einem weiteren Arbeitsgang hinreichend tiefgängig auf- und umgebrochen werden. Bevorzugte Aufund Umbruchtiefen dieser Bodenoberfläche liegen bei wenigstens 15 cm, bevorzugt sind allerdings entsprechende Arbeitstiefen im Bereich =/> etwa 30 cm. Es ist sofort verständlich: Landwirtschaftliche Arbeitsgeräte wie Eggen, insbesondere aber der Einsatz von Bodenpflügen ermöglichen die technologisch einfache Erfüllung dieser erfindungsgemäß geforderten Voraussetzung zur Modifizierung der zu sanierenden Erdreichoberfläche. Es kann dabei weiterhin bevorzugt sein, größere Auf- und Umbruchtiefen der Bodenoberfläche sicherzustellen, wobei hier entsprechende Maßzahlen von etwa 50 bis 80 cm oder auch noch darüber bevorzugt sein können. Auch hier stellt die heute zur Verfügung stehende Technologie des sogenannten Grubberns die technisch einfache Möglichkeit der Erfüllung solcher Voraussetzungen sicher.

Das hier dargestellte mechanische Aufreißen der Bodenoberfläche führt in mehrfacher Weise zu einer Optimierung der erfindungsgemäß durch biologische Sanierung einzustellende Reinigungsergebnisse. Das auf- und umgebrochene Erdmaterial wird dem verstärkten Luftzutritt zugänglich, bestehende Versickerungskanäle für Regenwasser in diesem oberen Bereich des Erdreichs sind zerstört, so daß eine gleichmäßige Durchfeuchtung dieser Erdreichschicht sichergestellt werden kann. Gleichzeitig handelt es sich dabei aber um die Schicht des sanierungsbedürftigen Erdreichs, in dem sich die besonders schwer abbaubaren vergleichsweise großräumigen MKW-Komponenten aufkonzentriert und verfestigt haben. Durch die dargestellte Umstrukturierung wird für die nachfolgende biologische Remediation eine völlig neue Ausgangssituation geschaffen.

Die erfindungsgemäße Lehre sieht weiterhin vor, in wenigstens einem Arbeitsgang das für die biologische Sanierung benötigte Nährstoffkonzentrat in die Erdreichoberfläche einzuführen. Dieses Nährstoffkonzentrat wird insbesondere in Form wäßrig verdünnter Lösungen, Emulsionen und/oder Dispersionen aufgetragen, wobei ein im wesentlichen gleichmäßig verteiltes Aufsprühen besonders zweckmäßig sein kann.

In einer abschließenden Verfahrensstufe des erfindungsgemäßen Verfahrens erfolgt eine intensive Nachwässerung. Hierdurch wird einerseits das aufgesprühte Nährstoffkonzentrat in dem umgebrochenen Bodenbereich gleichmäßig verteilt, zum anderen wird vorzugsweise die Möglichkeit geschaffen, daß Anteile des Nährstoffkonzentrates auch weiter nach unten in den durch die mechanische Aufarbeitung nicht betroffenen Bodenbereich absinken.

Die hier dargestellte Sequenz von Verfahrensschritten zeichnet sich insbesondere dadurch aus, daß die Kombination der Arbeitsschritte zu (b1) und (b2) in vielgestaltigen Ausführungsformen 1- und mehrfach eingesetzt werden können. Besonders wichtige Kombinationen dieser Arbeitsschritte zu (b1) und (b2) sind durch eine der nachfolgenden Sequenzen gekennzeichnet:

(b1) ⇒ (b2), d.h., hier wird erst die zu sanierende Bodenoberfläche aufgebrochen und erst nachfolgend das Nährstoffkonzentrat eingetragen. Ebenso ist es aber auch möglich im Sinne (b2) ⇒ (b1) zu arbeiten, d.h. zunächst auf den zu sanierenden Boden auf die noch unbearbeitete Oberfläche das Nährstoffkonzentrat aufzutragen und erst jetzt die mechanische Bearbeitung der Bodenoberfläche vorzunehmen.

In wichtigen weiteren Ausführungsformen werden entweder der Arbeitsschritt zu (b1) und/oder der Arbeitsschritt zu (b2) mehrfach, bevorzugt zweimal eingesetzt. Hieraus ergeben sich dann die weiterhin möglichen und gegebenenfalls bevorzugten Arbeitskombinationen für den Einsatz dieser Schritte zu (b1) und (b2) im Sinne der Sequenzen (b1) ⇒ (b2) ⇒ (b1) oder (b2) ⇒ (b1) ⇒ (b2).

Die Wässerungsstufen zu (a) und (c) können in der erfindungsgemäßen Lehre 1- und/oder mehrstufig ausgebildet sein. Die Arbeitsstufe (a) kann wie zuvor schon angegeben bei Vorliegen eines regennaßen Bodens entfallen. Ebenso kann eine Nachwässerung dann unnötig werden, wenn der Vorgang des Auftrags der Nährstoffkombination gem. (b2) unmittelbar vor oder während einer Regenphase stattfindet.

Wenn auch die Einzelheiten der erfindungsgemäßen mehrstufigen Arbeitsweise im jeweils betroffenen Fall durch die bestimmte Bodenkonstitution und/oder das Ausmaß der MKW-Belastung beeinflußt werden, so gilt doch weiterhin allgemein: Die ein- oder mehrfache Wiederholung wenigstens des Eintrags des Nährstoffkonzentrats für die biologische Sanierung unter geeigneten Feuchtebedingungen des zu behandelnden Bodens und insbesondere auch das nachfolgende 1- oder mehrstufige Nachspülen gem. der Arbeitsmaßnahme (c) sind zweckmäßig und bevorzugt. Der Zeitraum zwischen den einzelnen Stufen des wiederholten Nährstoffeintrags ist den jeweils vorgegebenen Bedingungen anzupassen. In der Regel beträgt er wenigstens etwa 1 bis 2 Wochen, wobei das Arbeiten in größeren Zeitabständen, beispielsweise im Bereich von 1 bis 6 Monaten, besonders geeignet sein kann.

Die Anzahl der einzusetzenden Wiederholungen der erfindungsgemäßen Sanierungsmaßnahme ist wiederum durch die im jeweiligen Einzelfall gegebenen Verhältnisse bestimmt. Die heute übliche regelmäßige Überprüfung des Schadstoffgehalts in den verschiedenen vertikalen Schichtstufen des verunreinigten Erdreiches gibt hier Auskunft ob und in welchem Ausmaß eine nochmalige Wiederholung der erfindungsgemäßen Maßnahme wünschenswert oder gar zweckmäßig ist. Im allgemeinen hat sich gezeigt, daß eine 1- bis 3fache Wiederholung dieser Maßnahmen ausreicht, auch tiefergehende Erdreichbereiche zu sanieren, sofern nicht Barriereschichten im Bodeninneren hier entgegenstehen.

Das Wachstum der MKW-verzehrenden geeigneten Mikroorganismenstämme unter aeroben Bedingungen wird bekanntlich auch durch den zur Verfügung stehenden Sauerstoff gesteuert. Hier können insbesondere in den tieferen Bereichen dann zusätzliche Maßnahmen notwendig werden, wenn auch hier einerseits beträchtliche MKW-Verschmutzungen vorliegen und zum anderen die Hinnahme dieser MKW-Verschmutzungen nicht toleriert werden kann. Die erfindungsgemäße Lehre kann sich hier einer Mehrzahl von heute bekannten technischen Maßnahmen bedienen, die für solche Sonderfälle Abhilfe schaffen. Geeignet ist beispielsweise die Maßnahme, den benötigten Sauerstoff in chemisch gebundener Form und dabei insbesondere als Wasserstoffperoxid in den benötigten Arbeitsbereich einzuführen. Von der Industrie sind entsprechende Handelsprodukte für den hier angesprochenen Einsatzzweck entwikkelt und werden frei angeboten, verwiesen wird beispielsweise auf das unter dem geschützten Handelsnamen "RENATox" vertriebene Produkt der Firma Degussa. Möglich ist aber auch die gezielte Zwangszuführung von Luftsauerstoff in die betroffenen Bodenbereiche, beispielsweise durch Anlegen von hinreichend tiefen Absaugschächten, aus denen die Gasphase abgepumpt und damit der Nachfluß von Luft durch die umgebende Bodenformation erzwungen wird. Verwiesen sei beispielsweise auf die Veröffentlichung des Bundesministeriums für Forschung und Technologie, Förderkennzeichen 1460505, "Technologieregister zur Sanierung von Altlasten", B. Böhnke und K. Pöppinghaus, Dezember 1990, dort insbesondere Seiten 31 und 32.

### Es sei in diesem Zusammenhang allerdings noch einmal klargestellt:

Die erfindungsgemäße Lehre ist nicht zwingend daran gebunden Altlasten auf MKW-Basis bis in beliebige Tiefen der betroffenen Bodenformationen zu entsorgen. Die Erfindung will primär den Bodenbereich erschließen und säubern, der durch übliche Lebensbeanspruchungen wie Pflanzenwachstum, Errichtung von Gebäuden und dergleichen, betroffen ist. In diesem Bereich vergleichsweise eingeschränkter Tiefe ist die möglichst einfache, dementsprechend kostengünstige und wirkungsvolle Reinigung notwendig und primäres Ziel der erfindungsgemäßen Lehre.

In diesem Zusammenhang sei auf eine weitere Möglichkeit verwiesen, die die Elemente des erfindungsgemäßen Handelns durch eine angepaßte (Zwischen)Pflege der zu reinigenden Erdreichflächen unterstützen:

So kann es beispielsweise erfindungsgemäß zweckmäßig sein, die aufgelokkerte Oberfläche des zu behandelnden Erdreichs mit tief wurzelnden Pflanzen zu bepflanzen, als typisches Beispiel sei hier der Anbau von Sonnenblumen genannt. Gleichzeitig damit oder auch anstelle dieses Beispiels können N-bildende Pflanzenansaaten auf die zu reinigende Erdreichoberfläche aufgebracht werden, Beispiele hierfür sind etwa Luzerne, Klee oder Ölrettich. Grundsätzlich eignen sich hier Vertreter aus der Klasse der Leguminosen.

Die nachfolgenden Ausführungen beschäftigen sich mit den erfindungsgemäß einsetzbaren Nährstoffen bzw. Nährstoffgemischen für die Aufzucht der MKW-verzehrenden Mikroorganismenstämme. Grundsätzlich gilt hier das allgemeine heute bekannte Fachwissen, wie es beispielsweise in der eingangs genannten DE-A-42 18 243 und der dort genannten Literatur beschrieben ist. Die sachliche Offenbarung dieser DE-A-42 18 243 zur Beschaffenheit geeigneter und insbesondere bevorzugter Nährstoffe bzw. Nährstoffgemische wird hiermit ausdrücklich auch zum Gegenstand der vorliegenden Erfindungsoffenbarung gemacht. Dementsprechend werden nachfolgend lediglich Auszüge aus ihrer Offenbarung hier noch einmal ausdrücklich angesprochen.

Zunächst einmal gilt auch für die erfindungsgemäße Lehre der in der Fachwelt bekannte Sachverhalt, daß die Nährstoffmedien sowohl auf Basis üblicher Düngemittel synthetischen und/oder natürlichen Ursprungs und damit insbesondere auf Basis wenigstens weitgehend hydrophiler Komponenten bestehen können. Wertstoffmischungen mit wenigstens anteilig hydrophoben Komponenten und insbesondere entsprechenden Verbindungen des P- und/oder des N können allerdings im hier betroffenen Anwendungsgebiet besondere Vorteile mit sich bringen. So können entsprechende Verbindungen des Phosphors (P) und des Stickstoffs (N) derart mit hydrophoben Molekülanteilen ausgerüstet sein, daß sich diese Moleküle den MKW-belasteten Erdbereichen anlagern. Der Vorteil einer solchen Interaktion zwischen der anzugreifenden Verschmutzung im Boden und dem Nährstoff bzw. Nährstoffgemisch für die Förderung des hier geeigneten Bakterienwachstums ist sofort einleuchtend: Das Wachstum der benötigten Bakterien und damit der Abbau der MKW-basierten Verschmutzungen wird hier optimal gefördert. In der Regel findet ein Auswaschen solcher hydrophobierten Anteile des Nährmittelgemisches nach unten in das Grundwasser nicht statt, während gegebenenfalls eingesetzte bzw. mitverwendete übliche anorganische und/oder organische Düngemittelkomponenten synthetischen und/oder natürlichen Ursprungs leicht einem solchen prinzipiell unerwünschten Auswaschprozeß unterliegen.

In an sich bekannter Weise werden diese hydrophobierte Wertstoffe enthaltenden Nährstoffkonzentrate zusammen mit Emulgier- und/oder Suspendierhilfen eingesetzt, wobei allerdings wenigstens anteilsweise diese Funktion auch einer oder mehreren Komponenten mit Nährstoffcharakter zukommen kann. Geeignete Zusatzkomponenten mit Emulgier- und/oder Suspendierwirkung sind die bekannten Netzhilfen auf Basis von Alkyloligoglukosiden, nähere Einzelheiten hierzu finden sich in der Offenbarung der zuvor benannten DE-A-42 18 243.

Erfindungsgemäß bevorzugte Nährstoffkonzentrate enthalten die Wasserund/oder Öl-Iöslichen Verbindungen des P und/oder des N in Abmischung mit weiteren Wasser- und/oder Öl-löslichen organischen Mischungskomponenten, denen wenigstens anteilsweise Nährstoffcharakter für das Wachstum von Kohlenwasserstoffverbindungen-verzehrenden Mikroorganismen zukommt. Besonders geeignet können Wasser-basierte Zubereitungen sein, die einen Ester der Phosphorsäure als Emulgator und P-Quelle sowie gewünschtenfalls eine oder mehrere Wasser-lösliche oder Wasser-dispergierbare N-Quellen enthalten. Es kann zweckmäßig sein Nährstoffkonzentrate einzusetzen, die 10 bis 40 Gew.-% Phosphorsäureester mit Emulgatorwirkung, 10 bis 40 Gew.-% der N-Quelle und zum Rest Wasser enthalten. Besonders bevorzugt können Nährstoffkonzentrate eingesetzt werden, die 20 bis 30 Gew.-% des Phosphorsäureesters mit Emulgatorwirkung, 15 bis 30 Gew.-% der N-Quelle und 0,5 bis 5 Gew.-% an nichtionischen Tensidverbindungen sowie zum Rest Wasser enthalten. Nährstoffkonzentrate die als Phosphorsäureester Phospholipide, Alkylphosphate und/oder Alkyletherphosphate enthalten, sind geeignete Vertreter im Sinne des erfindungsgemäßen Handelns. Die N-Quelle liegt in der Regel in Form anorganisch und/oder organisch gebundenen Stickstoffs vor, wobei Harnstoff als N-Quelle bevorzugt sein kann, der insbesondere in Mengen von etwa 10 bis 50 Gew.-% in der Nährstoffmischung zugegen sein kann. Als nichtionische Tensidverbindungen kommen neben den zuvor genannten Alkylglykosid-Verbindungen - hergestellt bevorzugt aus geradkettigen Fettalkoholen mit 8 bis 24 C-Atomen und Mono- und/oder Oligoglukosiden - weiterhin Zuckerpartialester von Monocarbonsäuren mit 8 bis 24 C-Atomen, Sorbitanester und/oder Biotenside biologischen Ursprungs in Betracht. Beispiele hierfür sind Sophoroselipid, Trehaloselipid und Lipopeptide.

Unter Bezugnahme auf die spezifische Offenbarung der DE-A-42 18 243 wird hier summarisch zu weiteren bevorzugten Elementen der Erfindung zusammengefaßt:

Besondere Bedeutung kann den eingesetzten Phosphorsäureestern zukommen, die zweckmäßigerweise so ausgewählt sind, das sie zum einen als P-Quelle dienen, gleichzeitig aber Emulgatorwirkung zeigen. Bevorzugte Phosphorsäureester werden von den aufzuzüchtenden Mikoorganismen aufgenommen. Unter den hier einsetzbaren Verbindungen kommen daher den Phospholipiden besondere Bedeutung zu. Phosphorlipide sind amphiphile Substanzen, die aus pflanzlichen oder tierischen Zellen gewonnen werden. Bevorzugte Phospholipide sind die Glycerophospholipide, die üblicherweise auch als Lecithin bezeichnet werden. Bekannte und einsetzbare Substanzen sind hier die Diacylphospholipide, Phosphatidylcholine, Phosphatidylethanolamine, Phosphatidylinositole, Phosphatidylserine, Phosphatidylglycerine, Phosphatidylglycerinphosphate, Diphosphatidylglycerin, N-Acylphosphatidylethanolamin und Phosphatidinsäure. Bevorzugt sind die Monoacylphospholipide, Lysophosphatidylcholine, Lysophosphatidylethanolamine, Lysophosphatidylinositole, Lysophosphatidylserine, Lysophosphatidylglycerole, Lysophosphatidylglycerophosphate, Lysodiphosphatidylglycerine, Lyso-n-acylphosphatidylethanolamine und Lysophosphatidinsäure. Wegen der Zugänglichkeit wird der Fachmann in erster Linie zu technisch verfügbaren Phosphatidylglyceriden greifen, die als pflanzliche oder tierische Lecithine und Zephaline im Handel sind. Diese Zubereitungen werden meist aus Ölen wie Maiskeimöl oder Baumwollsaatöl oder Sojaöl gewonnen. Im Rahmen der Erfindung bevorzugt sind die enzymatisch hydrolisierten Glycerophospholipide (enzymatisch hydrolisiertes Lecithin), die aufgrund der Abspaltung eines Fettsäurerestes einen hydrophilieren Charakter aufweisen. Ausgenommen sind dabei lediglich solche Produkte, die durch die enzymatische Hydrolyse ihren Phosphorsäurerest verloren haben.

Neben oder anstelle der genannten Phospholipide können als P-Quelle der Nährstoffkonzentrate auch Partialester der Phosphorsäure mit Fettalkoholen und dabei insbesondere mit geradkettigen Fettalkoholen eingesetzt werden. Zu Einzelheiten s. die Offenbarung der DE-A-42 18 243.

Als N-Quelle enthalten die Nährstoffkonzentrate entsprechende Verbindungen in Form anorganischer und/oder organisch gebundenen Stickstoffs. Bevorzugt können N-Quellen sein, die nur organisch gebundenen Stickstoff enthalten. Anorganische Salze wie Alkalinitrat oder -nitrit oder auch Amoniumsalze sind geeignete N-Quellen. Organische N-Quellen sind beispielsweise Aminocarbonsäuren, etwa von der Art der Glutaminsäure. Als besonders leicht zugänglich für die Steuerung und Begünstigung des Mikroorganismenwachstums hat sich Harnstoff erwiesen, der gleichzeitig einen Konservierungseffekt gegen nicht gewünschten mikrobiellen Befall erzielt, so daß die Nährstoffkonzentrate eine hohe Lagerstabilität aufweisen. Werden als P-Quelle Phospholipide verwendet, so kann auf die Mitverwendung einer zusätzlichen N-Quelle verzichtet werden, da die hier angesprochene Stoffklasse sowohl P als auch N enthält.

Zu den gegebenenfalls mitzuverwendenden Mischungskomponenten mit Tensidwirkung vgl. die zuvor gemachten Angaben, insbesondere zu den Alkylglykosidverbindungen, sowie die spezielle Offenbarung der DE-A-42 18 243. Die Tensidverbindungen liegen üblicherweise in Mengen von 0,5 bis 5 Gew.-% im Nährstoffkonzentrat vor. In der Regel reichen Tensidmengen bis etwa 1 Gew.-% aus um die beabsichtigte Anstoßstimulation des Mikroorganismenwachstums auszulösen und zu begünstigen. So können Tenside auf Basis der Alkylglykosidverbindungen etwa im Bereich von 0,5 bis 1 Gew.-% - bezogen auf Gesamtmischung - zur wirkungsvollen Verbesserung des Mikroorganismenwachstums Verwendung finden. Zu weiteren Einzelheiten, insbesondere geeigneter Arbeitsparameter wird auf die nun bereits mehrfach genannte Deutsche Druckschrift verwiesen.

Die Nährstoffkonzentrate werden in der üblichen Anwendung bevorzugt mit Wasser auf das 10 bis 50fache verdünnt und 1- oder mehrfach in Mengen von 5 bis 200 mg pro g Verunreinigung in die kontaminierten Bodenflächen eingetragen.

### Beispiele

Vier Versuchsfelder á ca. 30 m² auf einer über Jahrzehnte durch Oberflächeneintrag mit Treibstoffen verunreinigten Liegenschaft wurden ausgewählt.

Die Testfelder bestanden bis in drei Meter Tiefe im wesentlichen aus gut durchlässigen Mittelsanden mit Feinsandanteilen. Die oberen 20 cm Bodenschicht waren humos. Der jahreszeitlich schwankende Grundwasserflurabstand lag im Mittel bei 2 Metern.

Über die Tiefenverteilung bis in den Grundwasserbereich ließen sich verschiedene Schadstoffkonzentrationshorizonte ermitteln, wobei die Zonen höchster Kontamination in einer Tiefe von 0,8 - 1,5 m lagen.

Für die Bodensanierung wurde folgendes Behandlungsraster gewählt:

| **Behandlung** | **Testfeld 1** | **Testfeld 2** | **Testfeld 3** | **Testfeld 4** |
|---|---|---|---|---|
| Vorbehandlung | Bewässerung | • Rodung des bestehenden Bewuchses • Bewässerung | Bewässerung | keine Behandlung |
| Düngemaßnahme | Nährstoffkonzentrat gem. DE-A-4218243 | Nährstoffkonzentrat gem. DE-A-4218243 | Düngung mit landwirtschaftlichem Mineraldünger | keine Behandlung |
| Einarbeitung | Pflügen | Pflügen | Pflügen | keine Behandlung |
| Nachbehandlung | Bewässerung | Bewässerung | Bewässerung | keine Behandlung |
| Wiederholung der Düngemaßnahme nach 6 Wochen | Nährstoffkonzentrat gem. DE-A-4218243 | Nährstoffkonzentrat gem. DE-A-4218243 | Düngung mit Mineraldünger | keine Behandlung |

Der natürliche biologische Abbau war zuvor über drei Jahre verfolgt worden und aufgrund der Nährstoffarmut des Bodens wie zu erwarten kaum noch fortschreitend (Testfeld 4). Zu Versuchsbeginn ergaben sich folgende Ausgangsbelastungen:

| **Belastungsgrade** | **Testfeld 1 in mg/kg** | **Testfeld 2 in mg/kg** | **Testfeld 3 in mg/kg** | **Testfeld 4 in mg/kg** |
|---|---|---|---|---|
| oberer Bodenhorizont | 2.300 | 1.300 | 2.300 | 800 |
| ungesättigter Bereich 0,2-0,8 m Tiefe (außerhalb des Grundwasserschwankungsbereichs) | 3.800 | 10.200 | 2.100 | 4.300 |
| ungesättigter Bereich 0,8 - 1,50 m Tiefe (außerhalb des Grundwasserschwankungsbereichs) | 3.700 | 20.000 | 4.000 | 10.200 |
| gesättigter Bereich (im Grundwasserschwankungsbereich) | 360 | 2.700 | 1.800 | 2.400 |

Nach einer Reaktionszeit von April bis August wurden folgende Schadstoffreduktionen ermittelt:

| **Belastungsgrade** | **Testfeld 1 in mg/kg** | **Testfeld 2 in mg/kg** | **Testfeld 3 in mg/kg** | **Testfeld 4 in mg/kg** |
|---|---|---|---|---|
| oberer Bodenhorizont Schadstoffreduktion um | 1.100 52% | 700 46% | 2.400 keine | 700 keine |
| ungesättigter Bereich 0,2-0,8 m Tiefe (außerhalb des Grundwasserschwankungsbereichs) | 1.800 53% | 10.200 keine | 2.300 keine | keine |
| ungesättigter Bereich 0,8 - 1,50 m Tiefe (außerhalb des Grundwasserschwankungsbereichs) | 2.600 30% | 13.800 31% | 4.100 keine | 10.200 keine |
| gesättigter Bereich (im Grundwasserschwankungsbereich) | 160 55% | 2.100 22% | 1.900 keine | 2.600 keine |

Die Ergebnisse zeigen, daß der natürliche Abbau durch Einsatz geeigneter Nährstoffe - wie dem in der DE-A-4218243 beschriebenen Wertstoffgemisch - wesentlich beschleunigt werden kann. Dieser Effekt ist wie zu erwarten besonders deutlich in der ungesättigten Bodenzone, aber auch in der gesättigten zufriedenstellend. Das Roden des bestehenden Bewuchses - in diesem Fall krautige Pflanzen - bringt keinen nennenswerten Vorteil und wird nur in unwegsamem Gelände empfohlen. Weiterhin zeigt sich, daß herkömmliche landwirtschaftliche Dünger für eine Anregung des Abbaus ungeeignet sind, da die Nährstoffe sehr schnell ausgewaschen werden.

## Patentansprüche

1. Verfahren zur vereinfachten biologischen Sanierung von Erdboden-Flächen praktisch beliebigen Ausmaßes, die mit Mineralöl-basierten Kohlenwasserstoffen (MKW) und dabei insbesondere entsprechenden Altlasten kontaminiert sind, durch Behandlung mit Nährstoffkonzentraten zur Stimulation und Wachstumshilfe für eine beschleunigte An- und Aufzucht von MKW-verzehrenden Mikroorganismen, Verfahren, bei dem man
(a) nach einer vorgängigen Durchwässerung
(b1) in wenigstens einem Arbeitsgang einerseits die zu sanierende Erdbodenfläche tiefgängig auf- und umbricht,
(b2) und andererseits ebenfalls in wenigstens einem Arbeitsgang das Nährstoffkonzentrat in Form einer wäßrig verdünnten Emulsion und/oder Dispersion aufträgt und dabei insbesondere im wesentlichen gleichmäßig verteilt aufsprüht und
(c) abschließend intensiv nachwässert.

2. Verfahren nach Anspruch 1, **dadurch gekennzeichnet, daß** man die Kombination der Arbeitsschritte (b1) und (b2) in der Form einer der nachfolgenden Sequenzen vornimmt:
(b1) ⇒ (b2); (b2) ⇒ (b1); (b1) ⇒ (b2) ⇒ (b1) oder (b2) ⇒ (b1) ⇒ (b2).

3. Verfahren nach Ansprüchen 1 und 2, **dadurch gekennzeichnet, daß** man in der(n) Arbeitsstufe(n) (b1) Auf- und Umbruchtiefen der Bodenoberfläche von wenigstens 15 cm, vorzugsweise =/> 30 cm, einstellt, wobei entsprechende Maßzahlen von 50 bis 80 cm bevorzugt sein können.

4. Verfahren nach Ansprüchen 1 bis 3, **dadurch gekennzeichnet, daß** die Arbeitsstufen der Wässerung zu (a) und (c) 1- und/oder mehrstufig ausgebildet sein können, wobei die Arbeitsstufe (a) bei Vorliegen eines regennaßen Bodens entfallen kann.

5. Verfahren nach Ansprüchen 1 bis 4, **dadurch gekennzeichnet, daß** die 1- und/oder mehrstufige Wässerung gem. Arbeitsstufe (c) derart intensiviert wird, daß wenigstens die Durchdringung des gem. (b1) gelokkerten Bodenbereiches mit dem aufgebrachten Nährstoffgemisch sichergestellt ist, bevorzugt jedoch Anteile des Mehrstoffgemisches auch in tieferliegende Bodenteile eingetragen werden.

6. Verfahren nach Ansprüchen 1 bis 5, **dadurch gekennzeichnet, daß** wenigstens die Arbeitsstufen (a), (b2) und (c) in größeren Zeitabständen wiederholt werden, wobei bevorzugte Zeitabstände im Bereich von 1 bis 6 Monaten liegen und eine wenigstens 1- bis 3fache Wiederholung zweckmäßig sein kann.

7. Verfahren nach Ansprüchen 1 bis 6, **dadurch gekennzeichnet, daß** die eingesetzten Nährstoffkomponenten und insbesondere die entsprechenden Verbindungen des Phosphors (P) und des Stickstoffs (N) derart mit hydrophoben Molekülanteilen ausgerüstet sind, daß sich diese Moleküle den MKW-belasteten Erdbereichen anlagern.

8. Verfahren nach Ansprüchen 1 bis 7, **dadurch gekennzeichnet, daß** die in Arbeitsstufe (b2) eingesetzten Nährstoffkonzentrate Emulgierund/oder Suspendierhilfen enthalten, wobei wenigstens anteilsweise diese Funktion auch einer oder mehreren Komponenten mit Nährstoffcharakter zukommen kann, bevorzugt aber auch wenigstens anteilsweise entsprechende Zusatzkomponenten im Nährstoffkonzentrat enthalten sind.

9. Verfahren nach Ansprüchen 1 bis 8, **dadurch gekennzeichnet, daß** Alkyloligoglukoside als Emulgier- und Netzhilfen im Nährstoffkonzentrat mitverwendet werden.

10. Verfahren nach Ansprüchen 1 bis 9, **dadurch gekennzeichnet, daß** Nährstoffkonzentrate zum Einsatz kommen, die Wasser- und/oder Öllösliche Verbindungen des Phosphors (P) und des Stickstoffs (N) in Abmischung mit weiteren Wasser- und/oder Öl-löslichen organischen Mischungskomponenten enthalten, denen wenigstens anteilsweise Nährstoffcharakter für das Wachstum von Kohlenwasserstoffverbindungen verzehrenden Mikroorganismen zukommt, wobei Wasser-basierte Zubereitungen bevorzugt sein können, die einen Ester der Phosphorsäure als Emulgator und P-Quelle sowie gewünschtenfalls eine oder mehrere Wasser-lösliche oder Wasser-dispergierbare N-Quellen enthalten.

11. Verfahren nach Ansprüchen 1 bis 10, **dadurch gekennzeichnet, daß** Nährstoffkonzentrate eingesetzt werden, die 10 bis 40 Gew.-% Phosphorsäure-ester mit Emulgatorwirkung, 10 bis 40 Gew.-% N-Quelle und zum Rest Wasser enthalten.

12. Verfahren nach Ansprüchen 1 bis 11, **dadurch gekennzeichnet, daß** Nährstoffkonzentrate eingesetzt werden, die 20 bis 30 Gew.-% des Phosphorsäureesters mit Emulgatorwirkung, 15 bis 30 Gew.-% N-Quelle und 0,5 bis 5 Gew.-% nichtionische Tensidverbindungen sowie zum Rest Wasser enthalten.

13. Verfahren nach Ansprüchen 1 bis 12, **dadurch gekennzeichnet, daß** Nährstoffkonzentrate zum Einsatz kommen, die als Phosphorsäureester Phospholipide, Alkylphosphate und/oder Alkyletherphosphate enthalten.

14. Verfahren nach Ansprüchen 1 bis 13, **dadurch gekennzeichnet, daß** in dem Nährstoffkonzentrat die N-Quelle in Form anorganisch und/oder organisch gebundenen Stickstoffs vorliegt, wobei Harnstoff als N-Quelle bevorzugt sein kann, der insbesondere in Mengen von etwa 10 bis 50 Gew.-% in der Nährstoffmischung zugegen ist.

15. Verfahren nach Ansprüchen 1 bis 14, **dadurch gekennzeichnet, daß** Nährstoffkonzentrate zum Einsatz kommen, die als nichtionische Tenside Alkylglykosidverbindungen, hergestellt aus bevorzugt geradkettigen Fettalkoholen mit 8 bis 24 C-Atomen und Mono- und/oder Oligoglykosiden, Zuckerpartialester von Monocarbonsäuren mit 8 bis 24 C-Atomen, Sorbitanester und/oder Biotenside biologischen Ursprungs wie Sophoroselipid, Trehaloselipid und Lipopeptide, enthalten.

16. Verfahren nach Ansprüchen 1 bis 15, **dadurch gekennzeichnet, daß** man die Nährstoffkonzentrate mit Wasser auf das 10- bis 50fache verdünnt und 1- oder mehrfach in Mengen von 5 mg bis 200 mg pro g Verunreinigung in die kontaminierten Bodenflächen einträgt.

17. Verfahren nach Ansprüchen 1 bis 16, **dadurch gekennzeichnet, daß** zusätzlich Konzentrate von Mikroorganismen auf die Bodenfläche aufgetragen werden, die zum Abbau von Kohlenwasserstoffverbindungen befähigt und dabei bevorzugt natürlichen Ursprungs sind.

18. Verfahren nach Ansprüchen 1 bis 17. **dadurch gekennzeichnet, daß** zusätzlich eine Zwangsbelüftung tiefer gelegener Bodenformationen und/oder der Eintrag gebundenen und im Erdboden freiwerdenden Sauerstoffs vorgesehen ist.

## Claims

1. A process for the simplified biological rehabilitation of land areas of virtually any size which are polluted by mineral-oil-based hydrocarbons (MHCs) and, in particular, by corresponding long-term contamination by treatment with nutrient concentrates for stimulation and growth promotion for the accelerated cultivation of MHC-consuming microorganisms, **characterized in that**,
(a) after preliminary moistening/watering where necessary,
(b1) on the one hand, the ground to be rehabilitated is broken up to a sufficient depth and turned over in at least one operation,
(b2) and on the other hand the nutrient concentrate is applied in the form of a water-diluted emulsion and/or dispersion, more particularly by spraying in substantially uniform distribution, again in at least one operation and
(c) the area thus treated is intensively watered.

2. A process as claimed in claim 1, **characterized in that** steps (b1) and (b2) are carried out in one of the following sequences: (b1) ⇒ (b2); (b2) ⇒ (b1); (b1) ⇒ (b2) ⇒ (b1) or (b2) ⇒ (b1) ⇒ (b2).

3. A process as claimed in claims 1 and 2, **characterized in that**, in step(s) (b1), the soil surface is broken up and turned over to a depth of at least 15 cm and preferably =/> 30 cm, depths of 50 to 80 cm being particularly preferred.

4. A process as claimed in claims 1 to 3, **characterized in that** watering steps (a) and (c) may be carried out in one and/or more stages, step (a) being redundant where the soil is wet with rain.

5. A process as claimed in claims 1 to 4, **characterized in that** watering in one and/or more stage in step (c) is intensified so that at least the soil layer loosened in step (b1) is penetrated by the nutrient mixture applied, although parts of the multicomponent mixture are preferably introduced into deeper soil layers.

6. A process as claimed in claims 1 to 5, **characterized in that** steps (a), (b2) and (c) at least are repeated at relatively long time intervals, preferred time intervals being 1 to 6 months and at least one to three repetitions being useful.

7. A process as claimed in claims 1 to 6, **characterized in that** the nutrient components used and, in particular, the corresponding compounds of phosphorus (P) and nitrogen (N) are provided with hydrophobic molecule components so that these molecules attach themselves to the MHC-polluted soil.

8. A process as claimed in claims 1 to 7, **characterized in that** the nutrient concentrates used in step (b2) contain emulsifying and/or suspending aids, the same function also being performed at least partly by one or more components of nutrient character, although corresponding added components are at least partly present in the nutrient concentrate.

9. A process as claimed in claims 1 to 8, **characterized in that** alkyl oligoglucosides are used as emulsifying and wetting aids in the nutrient concentrate.

10. A process as claimed in claims 1 to 9, **characterized in that** the nutrient concentrates used contain water- and/or oil-soluble compounds of phosphorus (P) and nitrogen (N) in admixture with other water- and/or oil-soluble organic mixture components which are at least partly nutrients for the growth of hydrocarbon-consuming microorganisms, water-based preparations containing an ester of phosphoric acid as emulsifier and P source and optionally one or more water-soluble or water-dispersible N sources being preferred.

11. A process as claimed in claims 1 to 10, **characterized in that** nutrient concentrates containing 10 to 40% by weight of emulsifying phosphoric acid esters, 10 to 40% by weight of N source and, for the rest, water are used.

12. A process as claimed in claims 1 to 11, **characterized in that** nutrient concentrates containing 20 to 30% by weight of the emulsifying phosphoric acid ester, 15 to 30% y weight of N source, 0.5 to 5% by weight of nonionic surfactant compounds and, for the rest water, are used.

13. A process as claimed in claims 1 to 12, **characterized in that** nutrient concentrates containing phospholipids, alkyl phosphates end/or alkylether phosphates as phosphoric said esters are used.

14. A process as claimed in claims 1 to 13, **characterized in that** the N source is present in the nutrient concentrate in the form of inorganically and/or organically combined nitrogen, urea being a preferred N source which may be present in the nutrient mixture in quantities of, in particular, about 10 to 50% by weight.

15. A process as claimed in claims 1 to 14, **characterized in that** nutrient concentrates containing alkyl glycoside compounds obtained from preferably straight-chain C₈₋₂₄ fatty alcohols and mono- and/or oligoglycosides, sugar partial esters of C₈₋₂₄ monocarboxylic acids, sorbitan esters and/or biosurfactants of biological origin, such as sophorose lipid, trehalose lipid and lipopeptides, as nonionic surfactants are used.

16. A process as claimed in claims 1 to 15, **characterized in that** the nutrient concentrates are diluted with water in a ratio of 10 to 50:1 and are introduced into the contaminated soil one or more times in quantities of 5 mg to 200 mg per g of contamination.

17. A process as claimed in claims 1 to 16, **characterized in that** concentrates of microorganisms which are capable of degrading hydrocarbon compounds and which are preferably of natural origin are additionally applied to the soil surface.

18. A process as claimed in claims 1 to 17, **characterized in that** it additionally comprises the forced aeration of deeper layers of soil and/or the introduction of combined oxygen which is released in the soil.

## Revendications

1. Procédé d'assainissement biologique simplifié de sois de terrain d'une étendue pratiquement quelconque, qui sont contaminés avec des hydrocarbures (MKW) à base d'huile minérale et ainsi en particulier avec des substances de rebut correspondantes, par traitement de concentrés de substances nutritives en vue de la stimulation et de l'aide à la croissance pour une poussée et une croissance accélérée de microorganismes qui consomment les MKW, procédé dans lequel :
(a) après une irrigation préalable,
(b1) d'une part on perce et on défonce en profondeur dans au moins une étape opératoire la surface du sol à assainir,
(b2) et d'autre part également en au moins une étape opératoire on applique le concentré de produit nutritif sous forme d'une émulsion et/ou d'une dispersion diluée par de l'eau, et on pulvérise de façon particulièrement répartie pour l'essentiel d'une manière homogène, et
(c) pour finir on poursuit le lavage d'une manière intensive.

2. Procédé selon la revendication 1,
**caractérisé en ce qu'**
on effectue la combinaison des séquences des étapes de travail (b1) et (b2) sous la forme d'une des séquences suivantes :
(b1) ⇒ (b2) ; (b2) ⇒ (b1) ; (b1) ⇒ (b2) ⇒ (b1) ou (b2) ⇒ (b1) ⇒ (b2)

3. Procédé selon les revendications 1 et 2,
**caractérisé en ce qu'**
on ajuste dans la (les) étape(s) de travail (b1), les profondeurs de perçage et de défoncement de la surface du sol d'au moins 15 cm, de préférence =/> 30 cm, pour lesquelles des cotes de 50 à 80 cm peuvent être préférées.

4. Procédé selon les revendications 1 à 3,
**caractérisé en ce que**
les étapes de travail de lavage pour a) et c) peuvent être formées en une ou en plusieurs étapes dans lequel l'étape de travail a) peut manquer lors de la présence d'un sol humide de pluie.

5. Procédé selon les revendications 1 à 4,
**caractérisé en ce que**
le lavage en une et/ou en plusieurs stades conformément à l'étape de travail c) est intensifié de façon à assurer au moins l'infiltration de la zone de sol rendue meuble conformément à b1) avec le mélange nutritif appliqué, mais que de préférence des fractions de mélange de plusieurs substances sont aussi introduites dans les parties de sol situées en profondeur.

6. Procédé selon les revendications 1 à 5,
**caractérisé en ce qu'**
au moins les étapes de travail (a), (b2) et (c) sont répétées dans des intervalles de temps plus grands pour lesquels des intervalles de temps préférés se situent dans la zone de 1 à 6 mois et une répétition d'au moins 1 à 3 fois peut être appropriée.

7. Procédé selon les revendications 1 à 6,
**caractérisé en ce que**
les composants nutritifs mis en oeuvre et en particulier les composés correspondants du phosphore (P) et de l'azote (N) sont pourvus de fractions de molécules hydrophobes afin que ces molécules s'additionnent aux zones de sol contaminées par les MKW.

8. Procédé selon les revendications 1 à 7,
**caractérisé en ce que**
les concentrés nutritifs mis en oeuvre dans l'étape de travail (b2), renferment des adjuvants de mise en émulsion et/ou de mise en suspension, cette fonction pouvant aussi revenir au moins partiellement à un ou plusieurs composants ayant un caractère nutritif, mais avec de préférence aussi des composants additifs correspondants contenus au moins partiellement dans le concentré nutritif.

9. Procédé selon les revendications 1 à 8,
**caractérisé en ce qu'**
on utilise conjointement des alkyloligoglucosides comme adjuvants d'émulsion et de mouillage dans un concentré nutritif.

10. Procédé selon les revendications 1 à 9,
**caractérisé en ce qu'**
on utilise les concentrés nutritifs qui contiennent des composés solubles dans l'eau et/ou dans l'huile, du phosphore (P) et de l'azote (N) en mélange avec d'autres composants de mélange organiques solubles dans l'eau et/ou dans l'huile, auxquels revient au moins partiellement un caractère nutritif pour la croissance de micro-organismes qui consomment des composés hydrocarbonés, en préférant des préparations à base d'eau qui renferment un ester d'acide phosphorique comme agent émulsionnant et comme source de P, ainsi que si désiré une ou plusieurs sources de N solubles dans l'eau ou dispersibles dans l'eau.

11. Procédé selon les revendications 1 à 10,
**caractérisé en ce qu'**
on met en oeuvre des concentrés nutritifs qui contiennent de 10 à 40 % d'ester d'acide phosphorique ayant une action d'agent émulsionnent, de 10 à 40 % de source de N et pour le reste de l'eau.

12. Procédé selon les revendications 1 à 11,
**caractérisé en ce qu'**
on met en oeuvre des concentrés nutritifs qui contiennent de 20 à 30 % en poids d'ester d'acide phosphorique ayant une action d'agent émulsionnant de 15 à 30 % en poids de source de N, et de 0,5 à 5 % en poids de composée tensioactifs non ioniques ainsi que pour le reste de l'eau.

13. Procédé selon les revendications 1 à 12,
**caractérisé en ce qu'**
on utilise des concentrés qui renferment comme esters d'acide phosphorique des phospholipides, des alkylphosphates et/ou des alkylétherphosphates.

14. Procédé selon les revendications 1 à 13,
**caractérisé en ce que**
dans le concentré nutritif, la source de N se présente sous forme d'azote liée d'une manière minérale et/ou organique, avec une préférence pour l'urée en tant que source de N, qui est présente en quantités d'environ 10 à 50 % en poids dans le mélange de substance nutritive.

15. Procédé selon les revendications 1 à 14,
**caractérisé en ce qu'**
on utilise des concentrés de substances nutritives, qui contiennent comme agents tensioactifs non ioniques, des composés d'alkyl glycoside produits à partir d'alcools gras de préférence à chaîne droite, ayant de 8 à 24 atomes de carbone et des mono et/ou oligoglycosides, des esters partiels de sucre d'acides monocarboxyliques ayant de 8 à 24 atomes de C, des esters de sorbitane et/ou des agents tensioactifs biologiques d'origine biologique comme un lipide de sophorose, un lipide de triladose et des lipopeptides.

16. Procédé selon les revendications 1 à 18,
**caractérisé en ce qu'**
on dilue les concentrés nutritifs avec de l'eau au dixième au 50^{ème} et on introduit une fois ou plusieurs fois en quantités allant de 5 mg à 200 mg par g de souillures dans les surfaces de sol contaminées.

17. Procédé selon les revendications 1 à 16,
**caractérisé en ce qu'**
on applique en supplément sur la surface du sol, des concentrés de micro-organismes qui sont aptes à la dégradation de composés hydrocarbonés et sont pour cela de préférence d'origine naturelle.

18. Procédé selon les revendications 1 à 17,
**caractérisé par**
en supplément une ventilation forcée des formations du sol situées profondément et/ou l'introduction d'oxygène lié et qui se libère dans le terrain.
